# EUROPEAN PATENT APPLICATION

(11) **EP 1 936 379 A1**
(43) Date of publication of application: **25.06.2008**
(21) Application number: 06026521.2
(22) Date of filing: 21.12.2006
(51) Int. Cl.: G01N 33/569, G01N 33/574

(54) **Method for the detection of active hpv infection in biological samples by a sandwich enzyme-linked immunosorbent assay and test kit for performing the method**

(71) Applicant: BUCHNER, Erwin, 65205 Wiesbaden (DE)
(72) Inventor: Gorbulev, Stanislav, Dr., 65510 Idstein-Eschenhahn (DE)

(57) **Abstract**

Disclosed is a test method for detecting human papillomavirus (HPV) active infection, comprising the steps of a) obtaining a biological sample, b) treating the sample in order to release any HPV capsid protein that might be present in the sample, c) contacting the sample from step b) with an immobilized binding agent specific for HPV capsid protein, whereby an amount of the HPV capsid protein is bound to the binding agent as a viral antigen, d) contacting the viral antigen bound to the binding agent with a labelled detection agent, yielding an amount of detection labelled viral antigen, e) contacting the detection-labelled viral antigen of step (d) with a signal-producing conjugate specific therefore, yielding an immobilized conjugate, f) contacting the immobilized conjugate with a signal producing substrate, thereby generating a signal; and g) measuring the signal generated and thereby detecting the presence of any detection-labelled viral antigen in the sample. Further disclosed is a test kit for performing the method, the test kit comprising an ELISA plate coated with an immobilized monoclonal or polyclonal antibody.

## Description

### Field of the Invention

The invention relates to a method for detecting a human papillomavirus (HPV) infection and a test kit for performing the method on body samples.

### Background of the Invention

Papilloma viruses infect epithelial cells of the skin and of mucous membranes, generally inducing benign tumors or warts. More than 70 variants of papilloma viruses have been described in humans. According to Lowy [1994] the variants are arranged in three classes:
1.) genital-mucosal
2.) non-genital and
3.) epidermiodysplasia verruciformis (EV)-specific

Several HPV serotypes are strongly associated with the development of malignant genital diseases. In fact, nearly 100 % of all cervical cancers and high-grade cervical lesions contain detectable amounts of HPV DNA. Genital HPV variants are further classified into low risk and high risk serotypes. The low risk serotypes, such as HPV 6 and HPV 11, are only occasionally associated with cervical malignancies. It is the high risk HPV serotypes which account for the majority of cervical cancers. HPV 16 is the prevalent serotype and is detected in 40 to 60 % of all cases, followed by HPV 18 which is found in 10 to 20 % of all cases. Of minor importance are HPV 31, HPV 33 and HPV 45. Genital HPV infection is likely to be the most prevalent sexually transmitted disease in the world. Individuals are often unaware of infection since visible symptoms can be easily overlooked. The onset of cervical cancer can be preceded by an incubation period of often more than 20 years after the initial infection.

Cervical cancer is the second-common cause of death among women worldwide. It has been calculated that there could be as many as about 500,000 incidences and about 350,000 deaths per annum worldwide. Once diagnosed, one in three women with cervical cancer will die from the disease.

The large number of cases shows the urgent need for a method for the early detection of HPV-induced cervical neoplasias. The diagnosis then allows a timely treatment of the malignant alterations of the cervix uteri.

Long-term clinical studies have not succeeded in completely elucidating how HPV becomes carcinogenic. However, perhaps the most important step in HPV induced tumor genesis is the integration of the viral DNA into the genome of the host cell, followed by expression of the early (E) viral proteins E6 and E7. It is generally accepted that these early proteins disrupt the cell cycle through and interaction with the cellular proteins pRB and p53. Repeated exposure and infection by high risk HPVs, particularly by HPV 16 or HPV 18, often aid in tumor genesis. Further, different genetic factors such as HLA status and changes in other tumor suppressor genes appear to affect clearance of HPV and its recognition by the immune system.

Recent data published by Griesser et al. [2004] also demonstrated the prognostic utility of HPV capsid protein detection in mild and moderate dysplasias. It was observed that major capsid protein L1 expression correlated with remission in mild and moderate dysplasias, whereas the absence of L1 caspsid protein correlated with a progression of the lesion. These observations have been attributed to the biological characteristics of the L1 capsid, which has been used by various biotechnology companies to develop a vaccine against HPV. Vaccination studies showed that the major capsid protein L1 is a very strong activator of the immune system. The subsequent remission of the dysplasia and the complete disappearance of the koilocytes can be monitored cytologically. However, if the immune system is not activated, the dysplasia may proceed.

At present, the Papanicolaou (Pap) smear test is a well known method for preparing and staining cells obtained from cervical scrapings in order to assess the risk of cervical cancer. In this test, the cell are examined microscopically by a cytologist for the presence or absence of indicators of precancerous or cancerous cells, such as variations in the size of the cell nuclei, presence of large vacuoles (koilocytes), abnormal nuclear/cytoplasmic ratios, and hyperchromic nuclei. Pap test results are frequently entered into a scoring algorithm which includes these observations, as well as the number and type of cells identified by Pap smear as "Atypical Squamous Cells of Undetermined Significance" (ASCUS). Pap test histologic scoring indicates the progression of cervical epithelia preneoplastic lesions by differentiating them into three grades with increasing clinical severity. Cervical intraepithelial neoplasia grade 1 (CIN I) is the lowest grade, followed by CIN II and III which is the last stage before cervical *carcinoma in situ.* A CIN I score has also been described cytologically as low grade squamous intraepithelial lesions (LSIL)-CIN II and III are considered as high grade squamous intraepithelial lesions (HSIL).

Due to the high false positive rate of Pap smear analysis, the current follow-up protocol in case of a positive test result is a retest. In recent studies such as the ACCUS LSIL Triage Study and subsequent analyses it was ultimately concluded that the available HPV reflux testing was overall more effective at diagnosing low and high grade cervical dysplasias, when compared with repeated Pap analysis, and was possibly even more cost-effective.

The existing methods for detecting HPV infections in body samples can be divided in two categories: 1.) testing by revealing the viral DNA by using the hybridization of the nucleic acid (with or without amplification), and 2.) immunological testing by identifying the viral proteins using immunocytochemical staining with virus-specific antibodies. Also genetic HPV testing methods have proven more effective that reflux tests, their diagnostic stand-alone value is limited by the high prevalence of HPV infection in patients who do not have lesions, and who will not develop any such lesions. In more than 80 % of the latent infections, which have no clinical relevance, the viral DNA disappears within one to two years and so-called "genetic clearance" occurs. The advantage of DNA testing is high sample throughput, since the test can be performed in a microtiter plate format, as well as with high sensitivity due to the increased accessibility of the viral DNA after cell lysis.

However, HPV DNA testing has been hampered by a high percentage of false positive results. This may lead to substantial psychological and physical strain for the patients, since neither appropriate vaccination nor therapy can be offered. Nevertheless, many hospitals or physicians with a large patient base have to rely on such methods, since for them it is the only viable approach to examine a large number of samples in a short time.

Another known method is to assay for the protein product using immunocytochemical testing. In this instance, the active virus in its active (productive) infection stage is assayed. This stage is usually accompanied by dysplastic changes of the cell morphology. Furthermore, only in the active infection stage the virus can be spread. As outlined above, the prognostic value of the immunocytochemical testing for major capsid protein L1 is limited and allows only a compilation of clinically relevant diagnosis data and provides indications for the most promising treatment. Further limitations are that the method is time-consuming, has a low sample throughput rate and requires a complex microscopic sample assessment. Nevertheless, the immunocytochemical method is often preferred by cytologists and pathologists having a small patient clientele.

Contrary thereto, an enzyme-linked immunosorbent assay (ELISA) technology, using antibodies for L1, has a high throughput rate, combined with a high clinical relevance and a good prognostic value, would provide a new tool, especially for hospitals and physicians having a large patient base.

### Summary of the Invention

The following definitions shall apply with respect to the present invention:
A biological sample is a specimen, which may contain detectable HPV capsid protein. The sample may be blood, sputum, saliva, urine, cerebral spinal fluid (CSF), lymph, cervical swab smears or material from biopsies.

Carcinoma and its pre-stages are defined as HPV-associated cancer and its preliminary stages, in particular carcinomas of the anogenital area, specifically cervical carcinoma.

A capsid protein is a protein which encases the virus, i.e. a viral capsid protein. In case of HPV it is the major capsid protein L1 and the minor capsid protein L2.

Capture antibody is an antibody which is immobilized on a solid-phase carrier, in particular on a microtiter plate or other test means, and is suitable to perform the affinity binding to a specific antigen.

A detection antibody is an antibody which recognizes the capture antibody, and shows a different epitope than the specific antigen. The detection antibody is typically conjugated to a signal molecule or complexed with a signal-producing enzyme.

An object of the present invention was thus to provide a simple, fast, easy-to-use and highly sensitive ELISA test for the immunocytochemical detection of active HPV-infection in body samples. The test should have a high sensitivity. It should allow automatization and thus high throughput rates.

A further object was to provide diagnostics, prognostics and theranostics (i.e. choice of therapeutic intervention) of HPV-associated diseases, in particular skin or anogenital diseases. The test should be able to detect very low levels of HPV capsid protein. It should allow the detection of high, low and specific serotypes of HPV capsid protein in body samples obtained by non-invasive procedures.

The foregoing objects have been achieved with a test method for detecting human papillomavirus (HPV) active infection, comprising the steps:
a) obtaining a biological sample;
b) treating the sample in order to release any HPV capsid protein that might be present in the sample;
c) contacting the sample from step b) with an immobilized binding agent specific for HPV capsid protein, whereby an amount of the HPV capsid protein is bound to the binding agent as a viral antigen;
d) contacting the viral antigen bound to the binding agent with a labelled detection agent, yielding an amount of detection labelled viral antigen;
e) contacting the detection-labelled viral antigen of step (d) with a signal-producing conjugate specific therefore, yielding an immobilized conjugate;
f) contacting the immobilized conjugate with a signal producing substrate, thereby generating a signal; and
g) measuring the signal generated and thereby detecting the presence of any detection-labelled viral antigen in the sample.

The viral antigen is preferably the major capsid protein L1 or the minor capsid protein L2 of HPV. In a preferred embodiment, the binding agent or the detection agents is an antibody which may be monoclonal or polyclonal.

The method can be performed with a test kit comprising an ELISA plate coated with an immobilized monoclonal antibody for the capture of HPV capsid protein and a detection antibody capable of binding to the captured HPV capsid protein. Non-specific interactions with the monoclonal antibody are removed by washing with a buffer-solution. In one embodiment, the detection antibody is a biotinylated detection antibody, which is in subsequently combined with a) a reporter enzyme and streptavidin, and b) a signal-producing substrate. In another embodiment it is a fluorescence-labelled detection antibody.

### Brief Description of the Drawings

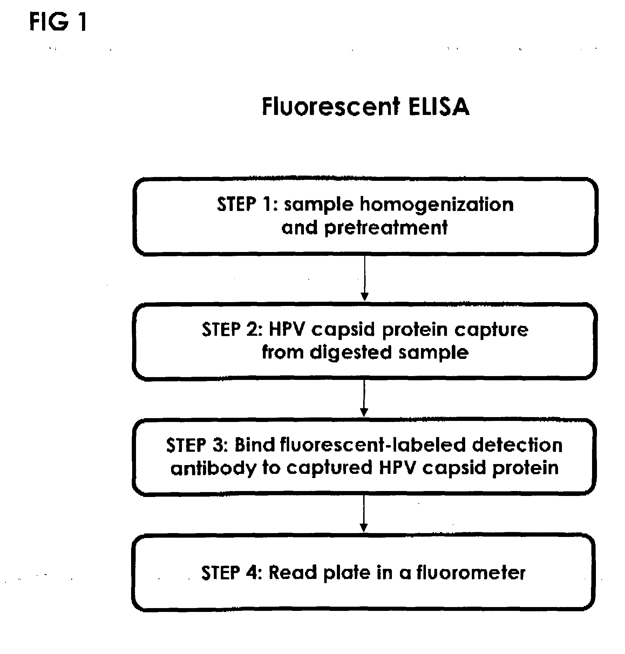

- Fig. 1: is a flowchart showing the step of the ELISA test according to the present invention, in which the presence of HPV capsid protein is indicated by a fluorescent signal.

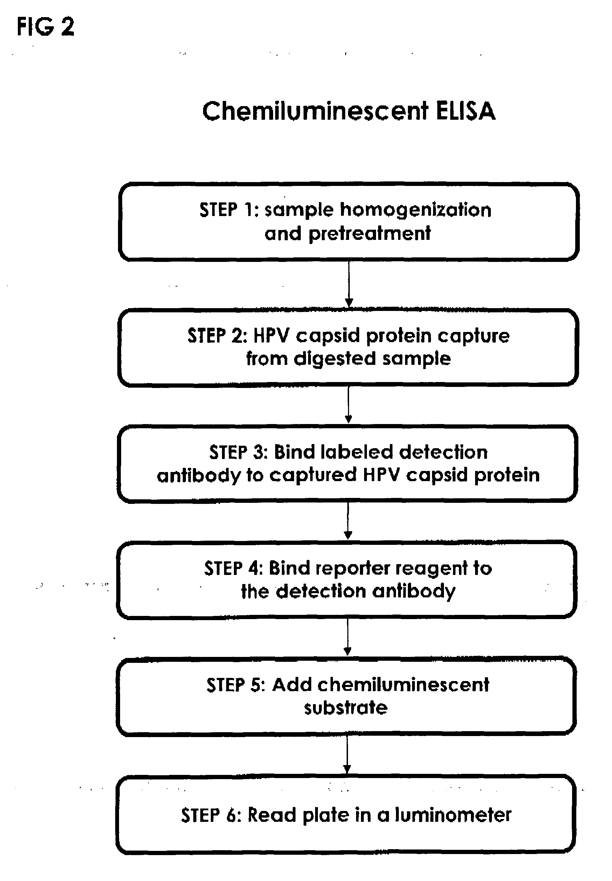

- Fig. 2: is a flowchart showing the steps of the ELISA test according to the present invention, in which the presence of HPV capsid protein is indicated by chemiluminescence.

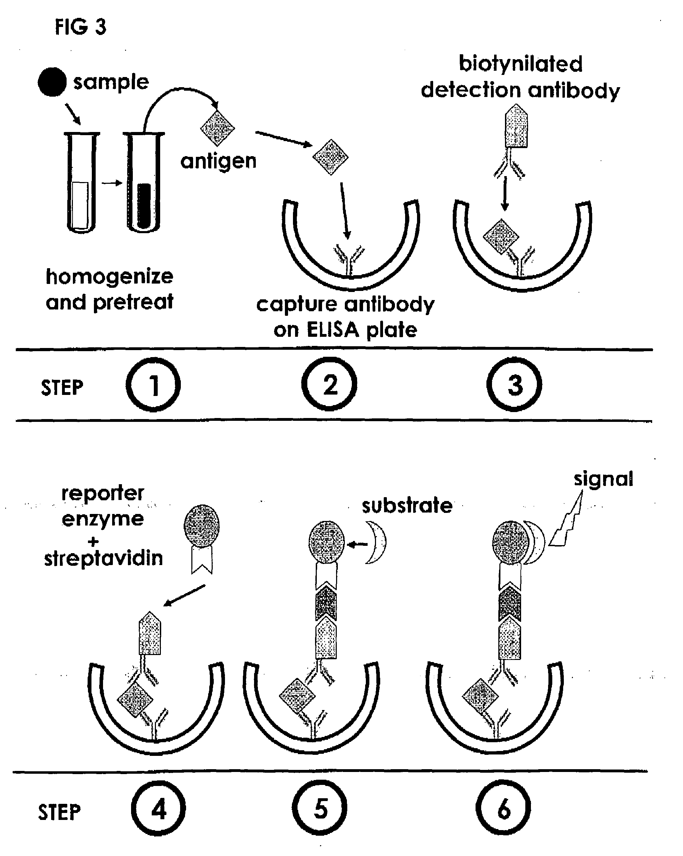

- Fig. 3: shows an embodiment of the method according to the present invention, wherein the presence of HPV capsid protein is indicated by a chemiluminescent signal.

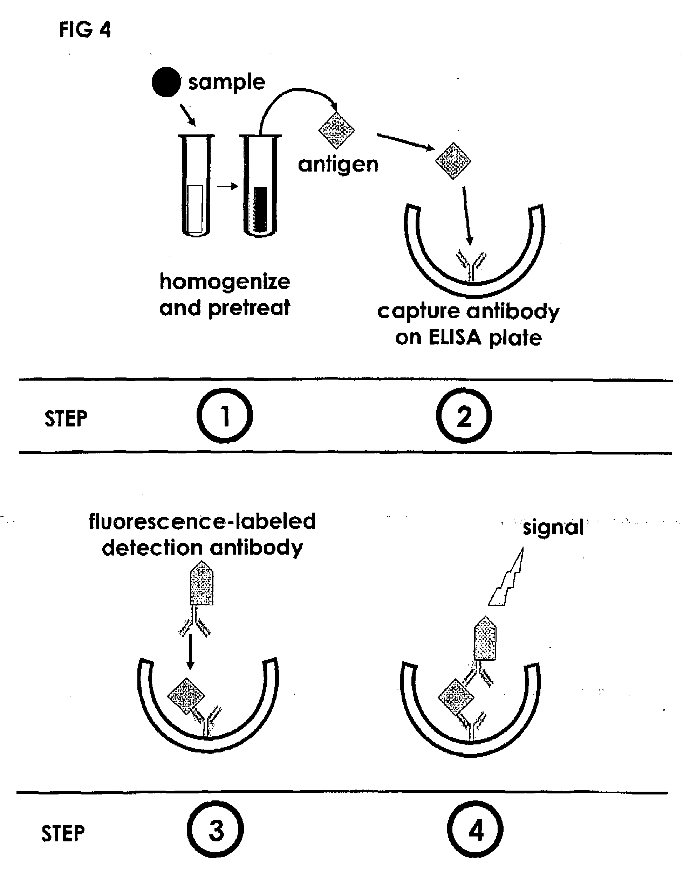

- Fig. 4: shows an embodiment of the method according to the present invention, wherein the presence of HPV capsid protein is indicated by fluorescence.

### Preferred Embodiments of the invention

The sample to be tested for the presence of HPV capsid protein is preferably a body sample from human tissue or body fluid. The sample is solubilized and homogenized, e.g. by incubating it with a pre-treatment mixture comprising a combination of enzymes and reagents for the liberation of HPV capsid protein and chemically reducing the disulfide bonds present within the HPV capsid. Incubation time and temperature can be adjusted as needed in order to achieve maximum sensitivity. The pre-treatment mixture preferably comprises lipolytic and nuclease enzymes as well as reducing reagents capable of liberating HPV capsid protein.

In a preferred embodiment, the HPV capsid protein is isolated from the pretreated sample, e.g. by centrifuging the sample in an ultracentrifuge and collecting the fractions (bands) comprising the HPV capsid protein. The isolated protein may be purified by methods known to persons skilled in the art, e.g. by dialyzing. After purification it is added to the wells of a fully blocked ELISA plate, In order to facilitate handling the isolated protein may be dissolved in a buffer.

The ELISA plate is usually a multi-well, high-protein binding, microtiter plate coated with an immobilized monoclonal or polyclonal antibody specifically interacting with HPV capsid protein. The monoclonal or polyclonal antibody is capable of capturing HPV capsid protein from the sample, if any such is present. It is an optimized protein which is normally combined with a buffer solution. Monoclonal antibodies are usually preferred as anti-HPV ligands, but other ligands may be used as well. The ELISA plate utilized in the present invention preferably is a multi-well microtiter plate that is opaque in order to eliminate "cross-talk". Cross-talk refers to light that comes from wells adjacent to the well being analyzed. The ELISA plate may be of any type known in the art, preferably a plate having a high-protein binding capacity. Regardless of the type of plate, is preferably coated with an immobilized anti-HPV ligand specific for HPV capsid protein. It is the anti-HPV ligand that captures any HPV capsid protein which may be present in the sample.

After adding the pretreated sample, a blocking agent is applied to the wells of the ELISA plate. The blocking agent is preferably a protein, such as gelatin, bovine serum albumin, non-fat dry-powdered milk or any optimized protein that does not interfere with the method of the present invention. The blocking agent binds to sites on the ELISA plate that are not already occupied by captured HPV capsid protein. The unoccupied binding sites must be fully blocked by the blocking agent in order to prevent non-specific binding of agents added in subsequent steps. In a preferred embodiment, the blocking agent is a mixture of high-purity casein, polyoxyethylene-sorbitan monolaurate and phosphate-buffered saline (PBS; pH 7.2).

In Step 3, a labelled detection antibody is added to the wells of the ELISA plate. It binds to the captured HPV capsid protein in the wells. Preferably it is an optimized protein and is applied in a buffer solution.

In a first embodiment a fluorescence-labelled detection antibody is added to the viral antigen bound to the immobilized monoclonal antibody. It is preferably a monoclonal antibody, more preferably 16L1-312F or 16L1-C22-7H, covalently attached to a fluorescent molecule, such as fluorescein. The fluorescent signal produced therefrom can be detected by any of known method using commercially available equipment. For instance, the ELISA well containing the fluorescent signal may be read in a microtiter plate fluorometer (fluorescence spectrometer). A fluorometer measures the amount of fluorescence emitted from a well that contains captured, detection-labelled HPV capsid protein. The intensity of the signal is directly proportional to the amount of detection-labelled HPV capsid protein in the well.

In the chemiluminescent embodiment preferably a biotinylated detection antibody is added to the viral antigen bound to the immobilized monoclonal antibody. Preferably, the detection antibody is a monoclonal antibody, such as 16L1-312F or 16L1-C22-7H. By the term "biotinylated" it is indicated that a biotin moiety is covalently attached to a protein or peptide for the purpose of reacting with another substance, such as alkaline phosphatase or streptavidin in a detection assay.

In the next step, a conjugate of streptavidin and a reporter enzyme, such as an alkaline phosphatase, or horseradish peroxidase is added to the sample treated with the biotinylated detection antibody. However, other reagents, such as streptavidin combined with calf intestinal alkaline phosphatase may also be used. When a chemiluminescent substrate is added, the conjugate provides the enzymatic activity necessary to produce a chemiluminescent signal which can be detected by any of the conventionally known methods. For example, the chemiluminescent signal can be measured by a microtiter plate luminometer. A luminometer measures the amount of light emitted from a well that contains the captured, detection-labelled HPV capsid protein. The intensity of the light produced is directly proportional to the amount of detection labelled HPV capsid protein in the well. Sensitivity can be further increased by employing enzyme polymers in the labelling step.

The present invention further includes a test kit that indicates the presence of HPV capsid protein by chemiluminescence and another test kit that indicates it by fluorescence. The chemiluminescence test kit comprises the following components:
- a sample pre-treatment mixture,
- an ELISA plate coated with an immobilized antibody,
- a labelled detection antibody,
- a chemiluminescent conjugate and
- a chemiluminescent substrate.

The ELISA plate is preferably coated with an immobilized monoclonal antibody, in particular with the monoclonal antibody 33L1 - 7. The labelled detection antibody is preferably a biotinylated form of the monoclonal antibody 16L1 - 312F. The chemiluminescent conjugate is preferably streptavidin and alkaline phosphatase, whereas the chemiluminescent substrate is preferably Sapphire ∥™ or Emerald ∥™.

The fluorescence test kit comprises the following components:
- a sample pre-treatment mixture,
- an ELISA plate coated with an immobilized antibody and
- a labelled detection antibody.

The ELISA plate is preferably a coated with an immobilized monoclonal antibody which acts as a capture antibody, more specifically with monoclonal antibody 33L1 - 7. The labelled detection antibody is preferably the monoclonal antibody 16L1 - 312F labelled with a fluorescence molecule. The fluorescence molecule is preferably fluorescein. The antibody 16L1-C22-7H may be used instead of 16L1 - 312F as the labelled detection antibody. In another embodiment, the monoclonal antibody 16L1-C22-7H is used as the capture antibody and 33L1 - 7 is used as the labelled detection antibody. In yet another embodiment, 16L1- 312F is used as the capture antibody and the monoclonal antibody 18L1-39-12D is used as the labelled detection antibody.

The following examples are intended to illustrate, not the limit the present invention. Percentages are percentages by weight, unless otherwise indicated or apparent from the context.

### Example 1: Detection of recombinant L1 protein from insect cells

infected insect cells were isolated by centrifugation at 1,000 g, washed and resuspended in a pH 7.5 buffer solution containing 20 mM Tris, 10 mM KCl and 1,5 mM MgCl₂. Subsequently they were lysed by homogenization in a Douncer, 50 strokes with a tight-fitted pestle. The cell nuclei were isolated in a centrifuge at 1,000 x g and resuspended in 5 ml phosphate-buffered saline (PBS), pH 7.3 per 5 x 10 ⁸ cells.

Virus-like particles (VLPs) were extracted by sonification. The density of the suspension was adjusted to 1.29 g/cm³ by adding caesium chloride. Next, the nuclear suspension was centrifuged an ultracentrifuge at 45,000 rpm for 15 hours at 4 °C in VTi 50 rotor. Fractions comprising the capsid bands were collected by needle aspiration. VLP-containing fractions having a density of 1.29 g/cm³ were pooled and dialysed three times against a citrate buffer (pH 6.0) and incubated for 20 min at 95 °C to retrieve the antigen.

The protein solution was diluted 100 fold with a PBS blocking buffer containing 0.05 % of Tween®-20 detergent. 5 % Bovine serum albumin was added to the blocking solution.

A 99 % pure protein solution containing 1 µg of protein per ml blocking buffer was used as a starting sample for the assay. The sample may be further diluted with the above-mentioned PBS blocking buffer, as needed, before being added to the 96-well plate.

The 96-well assay plate was then coated with the capture antibody 33L1 - 7 by adding 2 µg to 10 µg of the monoclonal antibody in PBS (pH 7.4).

Next, 100 µl of the protein sample diluted in the blocking buffer ware added to each well of the 96-well assay plate. The plate was subsequently incubated for 1 h at room temperature. After ten times washing the 96-well plate with PBST (= PBS + 0.05 % Tween-20; Tween® 20 is polyoxyethylenesorbitan monolaurate) in an automatic plate washer, 100 µl of biotin-labelled detection antibody 16L1-312F, diluted in blocking buffer, were added to the wells. The plate was then incubated for 1 hour at room temperature. Thereafter, the plate was again washed ten times with PBST in an automatic washer. Then, 100 µl of streptavidin-conjugated phosphatase were added to the wells, followed by another 1 h incubation at room temperature.

After the incubation period, the assay plate was again ten times washed with PBST in an automatic washer. The wells were washed with an alkaline phosphatase buffer containing 20 mM Tris (ph 9.7) and 1 mM MgCl₂. In the next step, 100 µl of an alkaline phosphatase substrate (CDP-Star® containing Emerald II™ enhancer from Tropix, Bedford, MA) were added to each well, and the plate was incubated, first for 20 min at room temperature, subsequently overnight at 4 °C. Luminescence was then determined using a Tropix 717 Microplate Luminometer from PE Applied Biosystems, Foster City, CA) at 542 nm. The luminescence measured was directly proportional to the amount of the recombinant HPV major capsid protein L1.

### Example 2: Detection of HPV L1 protein form tissue specimens

The sample was fresh tissue obtained during surgery, snap-frozen in liquid nitrogen. Minimum size of tissue specimens was 0.2 g. Pathology reports that accompanied each specimen showed the diagnosis.

Fresh cervical tissue specimens, ranging in size from about 0.2 to 1.0 g, obtained from 20 different patients, were minced into small pieces (1 mm³ or less), homogenized with a Teflon pestle on ice and washed once with PBS. A nuclear fraction was prepared by resuspending the cells in 10 ml of HEPES buffer (10 mM N-(2-hydroxy-ethyl)-piperazine-N'-2-ethanesulfonic acid, 10 mM KCI, 1.5 mM MgCl₂; pH 7.9) containing a protease inhibitor from Boehringer Mannheim. The samples were incubated on ice for 10 min.

Cells were lysed by homogenization in a Douncer, 50 strokes with tight-fitted pestle. Nuclei were collected by centrifugation at 2,000 x g for 10 min. They were resuspended in 2 ml of PBS and sonicated for 2 min, then 2.1 g of caesium chloride was added in order to adjust the density to 1.29 g/cm³. The suspension was placed into the tubes of an ultracentrifuge and centrifuged at 45,000 rpm for 36 h at 4 °C in a SW55 rotor. Fractions comprising the capsid bands were collected by needle aspiration.

The thus collected fractions were dialyzed against a citrate buffer (pH 6.0), the citrate buffer being changed twice, and then incubated 20 min at 95 °C to retrieve the antigen. The thus obtained protein solution was diluted 100-fold with the PBST-blocking buffer. 5 % Bovine serum albumin war added to the blocking solution.

A 96-well assay plate was coated with the capture antibody 33L1 - 7 by adding 2 µg 10 µg of the monoclonal antibody in PBS (pH 7.4). Next, 100: µl of the protein sample, diluted in blocking buffer, were added to each of the 96 wells. The plate was then incubated for 1 h at room temperature. After incubation, the plate was washed 10 times with PBST in an automatic plate washer. Subsequently, 100 µl of biotin-labelled detection antibody 16L1-312F, diluted in blocking buffer, were added to the wells and incubated for 1 h at room temperature. Subsequent to this incubation step, the assay plate was washed again 10 times with PBST in an automatic plate washer. After washing, 100 µl of streptavidin-conjugated alkaline phosphatase were added to the wells and the plate was incubated again for 1 h at room temperature. In the next step, the plate was washed again 10 times with PBST in an automatic plate washer, using as a washing liquid an alkaline phosphatase buffer containing 20 mM Tris (pH 9.7) and 1 mM MgCl₂. 100 µl of an alkaline phosphatase substrate (CDP®-Star containing Emerald II™ enhancer from Tropix) were added to each well, followed by another incubation, first for 20 min at room temperature and subsequently overnight at 4 °C. Luminescence was determined using a Tropix TR717 Microplate Luminometer from PE Applied Biosystems) at 542 nm. The luminescence was directly proportional to the amount of the recombinant major capsid protein L1.

## Claims

1. A test method for detecting human papillomavirus (HPV) active infection, comprising the steps of
a) obtaining a biological sample;
b) treating the sample in order to release any HPV capsid protein that might be present in the sample;
c) contacting the sample from step b) with an immobilized binding agent specific for HPV capsid protein, whereby an amount of the HPV capsid protein is bound to the binding agent as a viral antigen;
d) contacting the viral antigen bound to the binding agent with a labelled detection agent, yielding an amount of detection labelled viral antigen;
e) contacting the detection-labelled viral antigen of step (d) with a signal-producing conjugate specific therefore, yielding an immobilized conjugate;
f) contacting the immobilized conjugate with a signal producing substrate, thereby generating a signal; and
g) measuring the signal generated and thereby detecting the presence of any detection-labelled viral antigen in the sample.

2. A method as claimed in claim 1, wherein the viral antigen is the major capsid protein L1 or the minor capsid protein L2,

3. A method as claimed in claim 1 or 2, wherein the binding agent or the detection agent is an antibody.

4. A method as claimed in claim 3, wherein the capture antibody is monoclonal or polyclonal.

5. A method as claimed in claim 3, wherein the detection antibody is monoclonal or polyclonal.

6. A method as claimed in claim 3, wherein the detection antibody is labelled with biotin.

7. A method as claimed in claim 3, wherein the detection antibody is labelled with a fluorescent dye.

8. A method as claimed in claim 3, wherein the detection antibody is labelled with horseradish peroxidase.

9. A method as claimed in claim 3, wherein the detection antibody is labelled with alkaline phosphatase.

10. A method as claimed in claim 8 or 9, wherein an enzyme polymer is used in the labelling step.

11. A method as claimed in claim 3, wherein the detection antibody is visualized by another antibody.

12. A chemiluminescence test kit for performing the method as claimed in any of claims 1 to 11, said test kit comprising
- a sample pre-treatment mixture,
- an ELISA plate coated with an immobilized antibody,
- a labelled detection antibody,
- a chemiluminescent conjugate and
- a chemiluminescent substrate.

13. A fluorescence test kit for performing the method as claimed in any of claims 1 to 11, said test kit comprising
- a sample pre-treatment mixture,
- an ELISA plate coated with an immobilized antibody and
- a labelled detection antibody.
